# EUROPEAN PATENT SPECIFICATION

(11) **EP 2 730 586 B1**
(45) Date of publication and mention of the grant of the patent: **11.04.2018**
(21) Application number: 12793726.6
(22) Date of filing: 28.02.2012
(51) Int. Cl.: A61K 35/30, A61K 35/36, A61K 35/54, A61K 38/02, C07K 2/00, A61K 8/97, A61Q 19/02, A61Q 19/08, A61Q 19/00, A61P 17/02

(54) **PROTEIN-POLYPEPTIDE COMPLEX OBTAINED FROM TISSUES OF HOOFED LIVESTOCK EMBRYOS, PROCESS FOR PREPARING SAME AND PHARMACEUTICAL COMPOSITION**
PROTEINPOLYPEPTIDKOMPLEX AUS GEWEBE VON HUFTIEREMBRYONEN, VERFAHREN ZU SEINER HERSTELLUNG UND PHARMAZEUTISCHE ZUSAMMENSETZUNG DARAUS
COMPLEXE DE PROTÉINE-POLYPEPTIDE PROVENANT DE TISSUS D'EMBRYONS D'ONGULÉS , PROCÉDÉ DE PRODUCTION ET COMPOSITION PHARMACEUTIQUE LE COMPRENANT

(30) Priority: 30.05.2011 RU 2011121685
(43) Date of publication of application: 14.05.2014
(73) Proprietor: Aktsionernoe Obschestvo "Pharm-Sintez", Moskow, 111024 (RU)
(72) Inventor: NAZARENKO, Anna Borisovna, Moscow 121614 (RU); SOKOLOV, Mikhail Anatolevich, Moscow 107150 (RU)
(74) Representative: Held, Stephan
(86) International application number: PCT/RU2012/000140
(87) International publication number: WO 2012/166005

(56) References cited:
- WO-A1-2009/088314
- WO-A1-2010/007620
- EA-A- 200 000 634
- RU-C1- 2 132 688
- RU-C1- 2 142 785
- US-A1- 2006 058 238

## Description

### Field

The invention relates to biologically active substance - protein/polypeptide complex (PPC), prepared from tissues of skin, cerebrum and spinal cord of hoofed farm embryos and intended as a drug substance in medical products stimulating physiological and reparative regeneration in treatment of skin diseases and traumatic injuries and for use in medicine production in aesthetic medicine, beauty therapy, and a process for its preparation.

Authorized abbreviations:
PPC - protein/polypeptide complex;
I - ionic strength of solution;
Pharmcomposition - pharmaceutical composition.

At present, by a steady increase of skin diseases of autoimmune, vascular, toxic, infectious, and traumatic origin, and iatrogenic complications related to the usage of "anti-aging" products, cosmetics, and care, there is no direct reparants specific for skin tissue. The purpose of this invention was to provide a biologically active substance - direct reparant, which has a tissue-specific regenerative effect on skin tissue.

The biologically active substance is a PPC with a molecular weight of its constituent protein/polypeptide components ranging from 5 to 200 kDa and the minimum content of medium molecular fraction within the range of 20 to 160 kDa not less than 80%, with a total protein concentration of 0.1 - 4.2 mg / ml, with the solution ultraviolet spectrum absorption peak at wavelength of 215 ± 5 nm within UV-visible range with the wavelength interval of 200 - 500 nm (picture 1) and the presence of specific bands at denaturing gel electrophoresis in 5% polyacrylamide gel (picture 2), and a pharmaceutically acceptable diluent comprising a buffer and excipients - high-molecular compounds belonging to preservatives, detergents, and solubilizers classes in sufficient concentrations. The PPC is obtained by ion-exchange chromatography of extracted homogenate filtrate of nerve and skin tissues of hoofed farm embryos of gestation period from the middle of the first third to the middle of the last third of pregnancy, in the presence of buffer, detergents, and solubilizers.

### Prior Art

A number of preparations are known of protein-peptide nature, made from of raw materials of animal origin, having regenerative activity and used for treatment of skin diseases. The closest from the point of view of the effect achieved include:
- peptidic placenta extract and the method for its [1], involving salt extraction of placenta homogenate with 10 mM sodium phosphate buffer pH - 7.0 in presence of 0.1 mM EDTA and Triton-100 with prior conditioning at +4°C for 3 hours and further phase of hormones removal from the eluent after centrifugation in presence of polyglucin and activated carbon, with temperature brought to +40° - +45°C and leaving for 4 hours at room temperature. The eluent obtained from the centrifugated mix contains 5 - 35 mg/ml protein, 2 - 5 mg/ml glycoproteids and 0.5 - 1 mg/ml Triton X100. It is important to note a rather high yield by the amount of proteins obtained. However, the proteins obtained in the extract of proteins not characterized by standard physical and chemical methods (weight, optical density, phoresis, etc.), but the presence of a significant amount of glycoproteids allow for an assumption of a high immunological and allergenic potency of the extract;
- a group of inventions named *Medicinal products based on embryo and a method for producing the same* [2], this method involves grinding and homogenization of embryos soft tissues in mixture with physiological saline solution. This method does not allow for standardizing the obtained mixture and absence of clear physical and chemical characteristics of the product - for parenteral administration due to the presence of cell wall components and cell structure membranes, which provides for its high immunological potency;
- a method for production of biologically active preparation based on embryonal tissues [3], involving homogenate hydrolysis for 2 to 42 days at +4 - +45°C with further thermal treatment at +60 - +120°C, separation of denatured substances and extraction from the hydrolyzate of an undefined thermostable end product with average molecular weight below 10 kDa with peptide content of 1 - 3 mg/ml and solid residue 14 - 20 mg/ml. This method results in the end product containing modified denatured hydrolyzates with a disturbed quarternary and tertiary structure and man active molecules dissipation products, which defines its low biological activity;
- a method for biological skin rejuvenation [4], involving intradermal and subcutaneous administration of dispersed bio-compatible material "Alloplant" [5] having an ability to attract the macrophage range cells with simulation of their maturation, skin cover acid-alkali rebalancing through sweat and oil glands functioning normalization, with metabolic processes change to prevalence of the processes that are characteristic of a physiologically younger tissue. This results in the ability to correct fibrous and degenerative-dystrophic changes in tissues (the ability of selective tissue growth in place of implanted biological material) with the increase of metabolic processes providing skin fibroarchitecture restoration. But lack of balanced growth and differentiation factors specific for skin tissue does not allow for achievement of a pronounced rejuvenating effect and stimulate physiological and reparative regeneration.
- skin care preparation "Garmoniya" [6] containing mammal (human) embryo extract and natural and chemical biologically active additives (geranium oil, coriander oil, plantain tincture, spring birch leaves extract, spring bird cherry leaves extract, aloe and swallowwort latexes, maize oil, propolis extract, palm oil, sea buckthorn oil and hen egg-yolk, glycerine, inorganic salts, 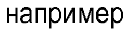 , such as Hanks' salts, dibasic sodium phosphate, citric acid, with the base to natural and chemical biologically active additives ratio being (0.59 - 5.3):1.0. Embryo extract is produced by homogenization and extraction in saline solution, ultracentrifugation with further supernatant filtration. The resulting extract contains heteromolecular fractions not characterized by standard physical and chemical methods, water-soluble denatured proteins, peptides, peptidoglycanes, non- standardizable lipoproteins, external application of which is quite immunogenic with a low biological effect;
- a method for producing a regenerating skin care cosmetic cream [7] containing, apart from growth factors including interleukine -2, erythropoietin, epidermal growth factor and leucocyte growth hormone, human and/or animal leucocyte metabolites with the following quantitative content of components (weight %): nutrient medium with growth factors and leucocyte metabolites - 0.7 - 8.5; natural and chemical biologically active substances - 7.0 - 27.0, obtained from the nutrient medium in which human and/or animal cells were incubated (e.g., leucocytes), in presence of somatotropin. This composition does not contain cytoplasmic, nuclear and membrane proteins and polypeptides, taking an important signaling part in cell regeneration and repair mechanisms, concentration of growth factors and hormones extracted by leucocytes is not optimal but is their waste and metabolic product, which significantly reduces the composition's biological activity, re-stimulating physiological regeneration and slightly stimulating repair of tissues, not containing tissue-specific factors for skin;
- there is a skin care composition known containing the following components: retinoid selected from a group including vitamin A alcohol formulation, vitamin A aldehyde formulation, retinyl acetate, retinyl palmitate and their mixture, oil phase having a low insaturation level, and a stabilizing system selected from a group including at least one oil-soluble antioxidant, chelating agent, licorice extract, hydroquinone, kojic acid, Gatuline A, micromerol, glutathione, magnesium phosphate L-ascorbyl-2, arbutin, placenta extract and their mixes [8]. But the known composition, when applied for a long term, can cause hypertension syndrome and does not make a satisfactory regenerating and rejuvenating effect.
- a skin care preparation based on growing mammal embryos, containing a gelatinous base and components based on growing mammal embryo extract, e.g. bovine embryos [9]. Extraction is made from the throat sweetbread, not from the whole animal embryo. The composition contains (10-30)% of the animal embryo extract and (90-70)% gelatinous base. The preparation is applied to the face and body in order to provide skin cells rejuvenation, wrinkle protection, skin strength enhancement and retardation of tissue aging. But this cosmetic shows system hormonal activity by influencing the entire organism, which has some counterindications, and is not effective enough from the point of view of regenerative action made to the skin due to absence of tissue-specific of growth factors and factors preventing from skin insenescence and ageing, preserving and increasing skin elasticity and good condition for a long time;
- a preparation normalizing skin tissues metabolism [10], including, apart from the fraction of low-molecular (?), associated with RNA proteins from cell nuclei and cytoplasm of any embryo tissues (for example tissues of pig brain, liver, thymus and spleen in different ratios), peptide fraction from pig fetal organs homogenate, produced by acid extraction of 0.15M HCl with heating on boiling water bath during 30 min and further neutralization with alkali to pH 7.0 and sediment centrifugation resulting in peptide concentration reaching 8 mg/ml. This method of deriving an end product allows for obtaining denatured low-molecular peptide fractions due to the effect made during acid extraction and boiling, which dramatically modifies the quarternary and tertiary structure of peptides not characterized by standard physical and chemical methods and, as a consequence, reduces biological and physiological activity of the products;
The closest to the subject group of inventions, including from the point of view of the methods for producing of the PPC, is the method for producing of a biologically active complex that provides neuroregeneration and neuroprotection [11]. This methods allows for obtaining a mixture of proteins and polypeptides from farm animals' embryo brain taken at a certain gestation period (from the beginning of the middle third to the middle of the last third of pregnancy) by the method of anion-exchange chromatography using for application to the equilibrated column a preliminarily filtered tissue homogenate supernatant with a buffer having pH 7.2 - 8.4; the target fractions are produced by separation of proteins and polypeptides associated with the carrier using 0.05M TRIS Glycine buffer solution as a mobile phase, containing 0.1 MM EDTA and 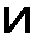 1M NaCl, increasing the concentration by stepwise gradient with a 5% increment. The above complex then undergoes ultrafiltration on a 5 kDa membrane and is desalinated. The target protein/polypeptide fraction characteristics are tested by UV spectrometry, gel-chromatography, denaturing polyacrylamide gel electrophoresis, and amino acid analysis methods. The above biologically active complex has an ultraviolet spectrum absorption peak at wavelength of 280 ± 5 nm and a constituent protein concentration from 5 to 110 kDa at least 95% of the total protein content. Usage of 1M NaCl within the mobile phase at the stage of anion-exchange chromatography for producing this complex generates an excessive ionic strength of the mobile phase equal to 1 moles per litre and inclusion of EDTA and TRIS into the composition increases it still more. I this connection, the obtained target fraction contains a great amount of acidic highly charged proteins and polypeptides, having in brain tissues cells mainly membrane localization performing mainly structural functions and only to a small extent regulatory functions not having tissue-specific nor even expressed biological activity. Further gradient increase of salt concentration with 5% increment still increases the presence of the said proteins, increasing the mixture's biological inactivity, by this reducing its activity. In production of this complex, usage of embryo brain (?) as raw material provides for the presence of tissue-specific proteins, regulating physiological and repair processes of preferentially nerve tissue, producing an unexpressed systemic effect, therefore, the biological activity scope is focused on correction and addressing of nerve system disorders and diseases.

### Disclosure of Invention

The cardinal object of the invention is production of an effective natural protein/polypeptide complex complying with current international safety requirements to pharmaceuticals.

The main goal set to the authors was production of a biologically active complex stimulating physiological and reparative regeneration of skin tissue for in treatment of skin diseases and traumatic injuries and for use in medicine production in aesthetic medicine, beauty therapy, and a pharmcomposition based on the PPC.

The goal set is achieved by the subject group of inventions providing that all conditions of the PPC and the pharmcomposition production are met.

The subject group of inventions includes the protein/polypeptide complex, a method for producing the same from nerve and skin tissues of hoofed farm embryos at a certain gestation stage, and a pharmcomposition based on this protein/polypeptide complex.

Now therefore, the goal set is achieved by the new protein/polypeptide complex (PPC), produced from nerve and skin tissues of hoofed farm embryos of gestation stage from the middle of the first third to the middle of the last third of pregnancy, including tissue-specific negatively charged faintly acid, neutral proteins and polypeptides qualified as growth and differentiation factors, signaling molecules, defining its biological and pharmacological activity, with molecular weights ranging from 5 to 200 kDa, therewith at least 70% of the total protein amount having molecular weights ranging from 20 to 160 kDa, having an indicative specific set of bands at denaturing gel electrophoresis («SDS-Page») in 5% polyacrylamide gel compared to the standard set of marker proteins having molecular weights ranging from 10 kDa to 250 kDa and a solution ultraviolet absorption peak at wavelength of 215±5 nm, at ultraviolet spectrum recorded at wavelengths ranging from 200 to 500 nm.

The goal set is also achieved with a new method for producing the above protein/polypeptide complex, in which nerve and skin tissues of hoofed farm embryos of gestation stage from the middle of the first third to the middle of the last third of pregnancy are stepwise:
Homogenized in a buffer at the buffer: tissue ratio of at least 1 : 0.5 with a simultaneous extraction in presence of nonionic detergents at minimum pH=5.2 and maximum pH=8.5, with further centrifugation at g ranging from 10 000 - 28 000 during 90 - 30 min;
   - the resulting supernatant is filtered;
   - the filtrate undergoes anion-exchange chromatography on a column with an anion-exchange carrier and separating proteins and polypeptides associated with the carrier, using for a mobile phase a buffer solution initially having ionic strength (I) not exceeding 0.1 mmol/L, increasing it smoothly or stepwise with 0.025 mmol/L increment and starting to collect target fractions with eluent ionic strength ranging from 0.125 to 0.15 mmol/L at pH from 5.2 to 8.5;
   - the resulting fractions are associated and undergo sterilization filtration.
Another invention of the subject group is a PPC-based pharmaceutical composition, prepared as a solution containing, as an active ingredient, a biologically active protein/polypeptide complex according to claim 1 in concentration of 0.05 - 2.0 mg/ml, obtained using the method according to claim 2, and a pharmaceutically acceptable diluent.

As a pharmaceutically acceptable diluent the pharmaceutical composition may contain a pharmacopoeial buffer and, excipients including high-molecular compounds, stabilizers, preservatives, and solubilizers.

The above pharmaceutical composition is intended for pharmaceutical and beauty industries, e.g. for external parenteral, intradermal, subcutaneous, intranasal or contact administration to mucous membranes and skin.
Below there is a brief description of the method for PPC production and the PPC itself.

### Preferred Embodiment

Now therefore, the protein/polypeptide complex is produced according to the following procedure (some operations are described with detailing not limiting the method):
- nerve and skin tissues of hoofed farm embryos of gestation stage from the middle of the first third to the middle of the last third of pregnancy are defrozen;
- homogenized in a buffer with a simultaneous extraction in presence of nonionic detergents at minimum pH=5.2 and maximum pH=8.5, with minimum ratio 1:0.5;
- the homogenate is separated from ballast substances by way of filtering through a close fabric with further centrifugation at g ranging from 10 000 - 28 000 during 90 - 30 min.,
   -- the supernatant is filtered through a membrane filter with pore diameter equal to 0.45 µm;
- the filtrate is applied to the equilibrated chromatographic column with an anion-exchange carrier, e.g. Toyopearl DEAE-650M;
   buffer solution as a mobile phase, having ionic strength not exceeding 0.1 mmol/L, increasing it 0.025 mmol/L increment and starting to collect target fractions with a buffer having ionic strength ranging from 0.125 to 0.15 mmol/L at pH from 5.2 to 8.5;
- the target fractions are associated and diluted with buffer, e.g. 0.05M glycine-NaOH at pH ranging from pH 5.2 to 8.5 and total protein concentration equal to 1.0 - 2.0 mg/ml;
- the solution obtained undergoes sterilization filtration through a membrane filter with maximum pore diameter 0.22 µm, and the total protein concentration is defined.

All preparatory processes and operations of obtaining the protein/polypeptide fraction are recommended to be performed at a temperature ranging from +2° to +8°C.

In order to identify the produced PPC, UV spectrometry, gel-chromatography, and polyacrylamide gel electrophoresis methods are used. The ultraviolet spectrum is recorded at wavelengths ranging from 200 to 500 nm: the absorption peak appears at wavelength of 215 ± 5 nm (Picture 1.). The constituent protein molecular weight is defined by denaturing gel electrophoresis («SDS-Page») in 5% polyacrylamide gel compared to the standard set of marker proteins having molecular weights ranging from 10 kDa to 250 kDa (Picture 2.). The complex has the isoelectric point value ranging from 4.2 to 8.4. The preparation includes proteins with molecular weights ranging from 5 to 200 kDa, among which at least 70% have molecular weights ranging from 20 to 160 kDa.

For producing the pharmcomposition, the PPC is diluted with a pharmaceutically acceptable diluent, e.g. 50mM glycine-phosphate, containing, if necessary, excipients, e.g., with concentrations of 0.5% mannitol, 0.0005% Polysorbate-80 and disodium hydrogen phosphate to pH not below 5.2 and not exceeding 8.5, with calculation of final total protein concentration (according to Lowry method) in the obtained solution within the range of 0.05 - 2.0 mg/ml. Repeated identification and filtration sterilization are performed.

In order to identify the produced PPC, UV spectrometry, gel-chromatography, and polyacrylamide gel electrophoresis methods are used. The ultraviolet spectrum is recorded at wavelengths ranging from 200 to 500 nm: the absorption peak appears at wavelength of 215 ± 5 nm (Picture 1.). The constituent protein molecular weight is defined by denaturing gel electrophoresis («SDS-Page») in 5% polyacrylamide gel compared to the standard set of marker proteins having molecular weights ranging from 10 kDa to 250 kDa (Picture 3). The preparation includes proteins with molecular weights ranging from 5 to 200 kDa, among which at least 70% have molecular weights ranging from 20 to 160 kDa.

Therefore, for the PPC extraction it is essential:
- to use as the raw material nerve and skin tissues of hoofed farm embryos of gestation stage from the end of the first third to the middle of the last third of pregnancy;
- that preservatives, detergents, and solubilizers were present in the buffer in sufficient concentrations;
- that buffer media pH was falling within the range of 5.2-8.5 at homogenization and anion-exchange chromatography stages;
- that during anion-exchange chromatography the ionic strength (I) of the eluent at the moment of separation of proteins and polypeptides associated with the carrier was not below 0.1 mmol/L, and the target fractions were collected with an eluent with ionic strength not exceeding 0.15 mmol/L;
- that in the described PPC the content of proteins with 20 kDa to 160 kDa molecular weights was at least 70% of the total protein content;
- that while indentifying the claimed PPC and performing denaturing gel electrophoresis («SDS-Page») in 5% polyacrylamide gel compared to the standard set of marker proteins, characteristic bands were present confirming and characterizing the complex protein/polypeptide constituents spectrum. In case of other production parameters, the protein/polypeptide component composition and its constituents ratios will differ.

The claimed biologically active protein/polypeptide complex is fundamentally different from any known biologically active substances produced from animal material by:
- raw material - nerve and skin tissues of farm embryos are used, of a certain gestation stage from the middle of the first third to the middle of the last third of pregnancy;
- methods for production -presence in the extracting (?) buffer detergents and solubilizers and absence of denaturing and degradable compounds - spirits, alkalies, acids, and ferments, allow for obtaining membrane, intercellular, cytoplasmic, nuclear proteins and polypeptides with preserved tertiary structure;
- usage within the mobile phase of millimolar cation concentrations in order to create a moderate ionic strength, by this allowing for obtaining a large amount of nerve tissue specific low charged proteins and polypeptides performing regulatory, signaling, and enzymatic functions, defining a pronounced regenerative and reparative biological tissue-specific activity;
- qualitative composition as a certain fraction of tissue (membranes, cellular, cytoplasmic, nuclear) hydrophilic, low charged proteins and polypeptides, with a concentration ratio of growth and differentiation factors and signaling molecules with molecular weights ranging from 5 kDa to 200 kDa, evolutionarily fixed in the ontogenesis;
- identification and standardization of the target fraction obtained - presence of specific bands, characteristic of this complex, in course of electrophoresis in 5% polyacrylamide gel;

- purity - the resulting protein/polypeptide complex does not contain admixtures such as lipids, peptidoglycanes, lipopolyproteins, carbohydrates, polysaccharides and other compounds;
- administration - parenteral (intradermal, subcutaneous), to mucous membranes (intranasal, subconjunctival) and skin;
- administration safety (absence of toxic, mutagenic, cancerogenic and allergenic effects);
- biological and pharmacological activity (reparative and regenerative) and its specificity (tissue- and organo-specificity).

The claimed pharmcomposition in form of PPC with a pharmaceutically acceptable diluent helps increase the PPC solution stability and its effect and shelf life.

The invention is illustrated with the following examples without limitation to its scope.

### Example 1. Method for producing the PPC.

### Production of a protein/polypeptide complex from nerve and skin tissues of sheep embryos

440 g of nerve and skin tissues of sheep embryos taken at gestation stage of 16 - 18 weeks is defrozen and homogenated in 1,200 ml of 0.05M TRIS glycine-phosphate buffer solution with pH 8.0, containing 1 mM of EDTA and 0.1% of mannitol, for 5 minutes at +4°C, the homogenate is separated from ballast substances by filtering through a close fabric with further centrifugation at g=10,000 for 90 min, separating the centrifuge effluent. The supernatant is filtered through a membrane filter with 0.45 µm pore diameter, the filtrate is applied to a 200 ml chromatographic column equilibrated with 4 volumes of 0.05 M glycine-phosphate buffer with pH=7.0, with an anion-exchange carrier Toyopearl DEAE-650M; the proteins associated with the carrier are separated by stepwise gradient, using the same buffer solution containing 0.05 M KCl (I = 0.1 mmol/l), increasing salt concentration with 0.025M (I = 0.025 mmol/l) increment and starting to collect target fractions with salt concentrations ranging from 0.075 M (I = 0.125 mmol/l) to 0.1 M (I = 0.15 mmol/l); the target fractions are associated and undergo ultrafiltration in a plant at backpressure not exceeding 8.0 kgf/cm² through materials with retention potential exceeding 200 kDa and diluted with 0.05M glycine-NaOH at pH=8.5 to the total protein concentration equal to 1.4 mg/ml; the solution obtained undergoes sterilization filtration through a membrane filter with maximum pore diameter 0.22 µm, and the total protein concentration is defined.

All preparatory processes and operations of obtaining the protein/polypeptide fraction are performed at a temperature ranging from +2° to +8°C.

The resulting protein/polypeptide complex characteristics:
- the preparation solution ultraviolet spectrum is recorded at wavelengths ranging from 200 to 500 nm: the absorption peak appears at wavelength of 215 ± 5 nm (Picture 1.);
- the constituent protein molecular weight is defined by denaturing gel electrophoresis («SDS-Page») in 5% polyacrylamide gel compared to the standard set of marker proteins having molecular weights ranging from 10 kDa to 250 kDa, the fraction includes proteins with molecular weights ranging from 5 to 200 kDa, among which 76% have molecular weights ranging from 20 to 160 kDa (Picture 2);
- the amount of proteins is defined according to Lowry method (without presedimentation) - 1.48 mg/ml.

### Example 2. Method for producing the PPC.

### Production of a protein/polypeptide complex from nerve and skin tissues of pig embryos

300 g of nerve and skin tissues of pig embryos taken at gestation stage of 16 - 18 weeks is defrozen and homogenated in buffer solution with pH=5.2, containing 50 mM of Tris-maleate, 1 mM of (EDTA) and 0.1% of mannitol, for 5 minutes at +2°C. The homogenate is separated from ballast substances by filtering through a close fabric with further centrifugation at g=28,000 for 30 min. The supernatant is filtered through a membrane filter with 0.45 µm pore diameter and applied to a 250 ml chromatographic column equilibrated with 4 volumes of maleate buffer with pH=5.2, with an anion-exchange carrier DEAE-Sepharose. The proteins associated with the carrier are separated by gradient of the maleate buffer solution with pH=5.2 containing 0.05M NaCl (I = 0,1 mmol/l), smoothly increasing salt concentration to 0.1 M (I = 0,15 mmol/l), starting to collect target fraction with salt concentrations ranging from 0.075 M (I = 0,125 mmol/l) to 0.1M (I = 0,15 mmol/l), the target fraction undergoes ultrafiltration in a plant at backpressure not exceeding 8.0 kgf/cm² through materials with retention potential exceeding 200 kDa and further undergoes sterilization filtration through a membrane filter with maximum pore diameter 0.22 µm.

In order to characterize the produced PPC, UV spectrometry, gel-chromatography, polyacrylamide gel electrophoresis, and amino acid analysis methods are used. The ultraviolet spectrum is recorded at wavelengths ranging from 200 to 500 nm: the absorption peak appears at wavelength of 215 ± 5 nm (Picture 3.). The constituent protein and polypeptide molecular weights are defined by denaturing gel electrophoresis («SDS-Page») in 5% polyacrylamide gel compared to the standard set of marker proteins having molecular weights ranging from 10 kDa to 250 kDa (Picture 4). The preparation includes proteins with molecular weights ranging from 5 to 200 kDa, among which 72% have molecular weights ranging from 20 to 160 kDa. The amount of proteins is defined according to Lowry method (without presedimentation) - 2.4 mg/ml.

### Example 3. Method for producing the pharmcomposition

The PPC solution produced according to Example 1 is diluted with 50 mM of glycine-phosphate buffer solution, then excipients are added: detergents, solubilizers, preservatives, and high-molecular compounds to the final PPC concentration of 0.05 mg/ml, mannitol - 0.5%, polysorbate-80 - 0.0005%, disodium hydrogen phosphate to pH=8.5 and Nipagin - 0.03%. The resulting pharmcomposition undergoes sterilization filtration through a membrane filter with 0.1 µm maximum pore diameter, is bottled to aseptic glass bottles and sealed.

In order to characterize the resulting pharmcomposition with PPC, UV spectrometry, gel-chromatography, polyacrylamide gel electrophoresis, and amino acid analysis methods are used. The ultraviolet spectrum is recorded at wavelengths ranging from 200 to 500 nm: the absorption peak appears at wavelength of 215 ± 5 nm (Picture 1). The constituent protein and polypeptide molecular weights are defined by denaturing gel electrophoresis («SDS-Page») in 5% polyacrylamide gel compared to the standard set of marker proteins having molecular weights ranging from 10 kDa to 250 kDa (Picture 2). The preparation includes proteins with molecular weights ranging from 5 to 200 kDa, among which 72% have molecular weights ranging from 20 to 160 kDa. The amount of proteins in the produced PPC is defined according to Lowry method (without presedimentation) - 1.55 mg/ml.

### Example 4. Method for producing the pharmcomposition

The PPC solution produced according to Example 2 is diluted with 50 mM of glycine-phosphate buffer solution, then excipients are added: detergents, solubilizers, preservatives, and high-molecular compounds to the final PPC concentration of 2 mg/ml, mannitol - 0.5%, polysorbate-80 - 0.0005%, benzalkonium chloride - 0.005% and sodium dihydrogen phosphate to pH=5.2. The resulting pharmcomposition undergoes sterilization filtration through a membrane filter with 0.1 µm maximum pore diameter, is bottled to aseptic glass bottles and sealed.

In order to characterize the resulting pharmcomposition with PPC, UV spectrometry, gel-chromatography, polyacrylamide gel electrophoresis, and amino acid analysis methods are used. The ultraviolet spectrum is recorded at wavelengths ranging from 200 to 500 nm: the absorption peak appears at wavelength of 215 ± 5 nm (Picture 3). The constituent protein and polypeptide molecular weights are defined by denaturing gel electrophoresis («SDS-Page») in 5% polyacrylamide gel compared to the standard set of marker proteins having molecular weights ranging from 10 kDa to 250 kDa (Picture 4). The preparation includes proteins with molecular weights ranging from 5 to 200 kDa, among which 72% have molecular weights ranging from 20 to 160 kDa. The amount of proteins in the produced PPC is defined according to Lowry method (without presedimentation) - 2 mg/ml.

### Example 5. Toxicity Study

The produced pharmcomposition properties were studied using common methods for experimental (non-clinical) study of pharmaceutical substances [11].

Toxicity studies of the original pharmcomposition in acute and subchronic experiments have been carried out. The presentation is a solution for injection: five 1 ml ampoules (0.1 mg/ml), five 2 ml ampoules (0.1 mg/ml), one 2 ml ampoule (0.2 mg/ml). The maximal recommended daily dose is 0.4 mg in case of intralumbar injection and 0.1 mg in case of intravenous injection.

### Experimental Conditions and Research Methods

The study objective was to define the original pharmcomposition tolerance, toxic, and lethal doses. The experiments were performed in BALB/C mice of both sexes, weighing 18-20 g, at intraperitoneal, subcutaneous, and intracutaneous introductions. The observation time was 14 days. The animals were taken from a nursery with passports. The mice were kept in standard conditions in plastic cages 10 animals in each, 10 in each group. The animals were fed with combined feed, fresh vegetables, farmer cheese. Water and food were freely available to the animals. The vivarium had artificial daylight. The air temperature was +18° - +20°C [14]. The maximum possible dose of the presentation for mice is 0.2 ml and it was 10 mg/kg, which exceeded the daily therapeutic dose by 600 times. The animals were withdrawn from the experiment by etherization, dissected and examined for internal organs condition.

### Acute Toxicity Study Results

*Mice.* The animals weighed 18 to 20 g. The introduced dose was 0.2 ml, which was 10 mg/kg. The number of animals within one group was 10. There were two groups: for intragastric and intraperitoneal introduction. The observation time was 14 days.

*Appearance.* By the end of the experiment, all animals looked healthy. The hair was thick, white. Animals from different groups did not differ in appearance.

*Behavior. The animals' behavior did not differ between the groups and from the norm.*

*Mortality.* In all three groups no fatal cases were recorded.

*Animals' weights.* During the entire observation period six check weighing had been performed. Sharp weight loss or gain had not been recorded.

*Internal organs.* The groups did not differ in internal organs condition. Modification of organs or organomegaly had not been recorded.

### Subacute (Subchronic) Toxicity Study

### Experimental Conditions and Research Methods

The original pharmcomposition toxicity study in subchronic experiment was performed in male and female Wistar rats with weights ranging from 200 to 250 g. The animals were taken from a nursery. The rats were kept in standard conditions in plastic cages 5 animals in each. They were fed with pelletized feed, fresh vegetables, farmer cheese. Water and food were freely available to the animals. The vivarium had artificial daylight. The air temperature was +18 - +20°C. Prior to the experiment, the animals were kept in quarantine for 10 days. A group included 10 animals (5 males and 5 females). The groups were divided as follows:
1 group (reference) intact animals
2 group - the original pharmcomposition T. 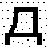 . intracutaneous introduction (π/κ)
3 group - the original pharmcomposition x 10 π/κ
4 group - the original pharmcomposition intraperitoneal introduction (B/B) T. 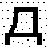 .;
5 group - the original pharmcomposition B/B x10.

Observation period: one month introduction, one month resorptive effect observation.

Toxicity was assessed against an observation checklist, including:
- survival rate and general appearance: (hair, eyes, ears, limbs, teeth);
- animals' condition and behavior (activity, gait, temperament, food acceptance);
- body weight variation (the rats were weighed prior to and after the experiment);
- physiological functions (breathing, salivation, urination, excretion).

Experimental animals' blood was taken for analysis prior to and at the end of the experiment:
periferic blood morphological composition (red blood cells, white blood cells, thrombocyte counts, Hb level); biochemical values (protein, urea, creatinine, glucose levels), activity of some plasma enzymes (aspartate- and alanine aminotransferase, alkaline phosphatase, lactate-dehydrogenase). Blood for hematologic study was taken from tail veins. Blood corpuscles were counted using an automatic blood analyzer Picoscale (Hungary). Hb level was defined by cyanmethemoglobin method. Biochemical value levels were measured with automated system. FP-901, «Labsystems» Finland. Upon completion of a chronic experiment, the animals were sacrificed using narcosis overdose for pathomorphological study of organs and tissues. The animals were dissected immediately upon their death, according to a full pathoanatomical scheme. For pathomorphological studies, tissue samples were gelatinated and cryostated to obtain sections up to 5 µm thick. The tissues then were fixed and stained with hematoxylin-eosin. Microscopical investigation was performed with an Opton microscope (Germany). The experimental data were compared to the reference data and analyzed using a statistic software.

### Macroscopic Examination Results

*Survival rate:* all experimental animals survived all introduction methods. Deaths were not recorded neither in experimental nor in reference groups.

*Behavior*: no significant behavior (hyper- or hypoactivity) or condition deviations in experimental animals compared to reference groups were observed. Myotonus was not marked with hypererethism.

*Appearance*: all animals were medium finish, did not suffer from exhaustion or obesity. Hair was smooth and glossy; hairslip or fragility were not detected. Corneal opacity, tearing or any pathological characteristics were not observed. Pinnae were pink with no crust, not inflamed, jerking was not observed. Teeth were of normal color, breaking was not observed.

*Functions:* experimental animals breathing, like that of reference animals, was quiet, of normal rhythm, unobstructed; salivation with no distress; urinary frequency, amount and color of urina fell within physiological range.

Clinical study: *body weight dynamics:* positive in all experimental groups. There was no significant difference between experimental and reference animals.

*Haematologic values:* in all groups Hb level, red blood cells, white blood cells, thrombocyte counts fell within physiological range.

*Biochemical study:* the peptide complex taken in test dose did not affect the total plasma protein in experimental animals, which is indicative that the preparation being studied does not adversely affect the protein generating liver function. In order to detect possible adversely affect on liver, in course of the chronic experiment, alkaline phosphatase, aspartate and alanine aminotransferase activity was defined in animals' blood plasma.

Data analysis showed no significant changes in the above plasma enzymes activity.

### Pathomorpholoaical data: macroscopic examination

At dissection, in animals of all groups the skin was clear, subcutaneous fat layer is moderate. Internal organs were allocated in a regular way; there were no free fluids in pleural and abdominal cavities. Tracheal and bronchial lumens were free, their mucosae clear, moist, glossy. Lung tissue in the experimental group was more intensely colored than with intact reference animals, but bore no oedema signs. Myocard section showed no pathological findings.

The tongue was clear. Oral and esophageal mucosae had no defects. Stomach and bowel bore no traces of irritation. Hepatomegaly was not observed. Renal capsule is easily detachable; medulla and cortical substance were well distinguished at section. The splenic capsule was smooth, grayish-brown at section, splenic pulp without scraping. Testes and ovaries were unremarkable. Thymus was grayish, with no hemorrhage. Thyroid dense with symmetrical portions. Cerebral meninges of moderate blood filling, moist, glossy. Brain substance had a symmetrical pattern at section. Tested animals organs weight indices had no significant difference from reference groups.

Microscopic Study: in all tested groups: in internal organs (liver, kidneys, lungs, heart, spleen, stomach, large and small intestine), internal secretion glands (thyroid, thymus, suprarenal capsules, pancreatic gland), reproductive organs (uterus, ovaries), lymph glands, cerebrum, skin, nose and throat mucosae pathomorphological changes were not detected.

Subchronic toxicity study in immature animals at intracutaneous introduction in immature animals.

Study was performed in three weeks old baby chinchilla rabbits weighing 300 to 500 g. The preparation was introduced subcutaneously once or twice daily by 0.5 ml (0.05 mg) for two weeks. The animals were taken from a nursery. The rabbits were kept in standard cages 1 animal in each. They were fed with pelletized feed and fresh vegetables. Water and food were freely available to the animals. The vivarium had artificial daylight. The air temperature was +18 - +20° C. There were 3 groups, 5 animals in each. The groups were divided as follows: 1 group (therapeutic dose) - pharmcomposition 0.1 mg/ml 0.5 ml introduced once daily; 2 group - pharmcomposition 0.1 mg/ml 0.5 ml introduced twice daily; 3 group - intact animals. Introduction period was 2 weeks.

Toxicity was assessed against an observation checklist: by survival rate, general appearance, animals' condition and behavior, body weight variation (the rabbits were weighed at the beginning and at the end of the experiment). In course of the experiment, the local irritant effect was defined by changes at visual and histological examination in skin, at sites of injection.

Prior to and at the end of the experiment, experimental animals were subjected to periferic blood morphological composition analysis (red blood cells, white blood cells, thrombocyte counts, Hb level); biochemical values (protein, urea, creatinine, glucose, total cholesterol and triglycerides levels), activity of some plasma enzymes (aspartate- and alanine aminotransferase, alkaline phosphatase, lactate-dehydrogenase). To define the said values, blood was taken from auricular veins in the amount of 1.5-2.0 ml. Blood corpuscles were counted using an automatic blood analyzer Picoscale (Hungary). Hb level was defined by hemiglobincyanide method. Biochemical value levels were measured with automated system. FP-901, «Labsystems» Finland.

Upon completion of a chronic experiment, the animals were sacrificed using narcosis overdose for pathomorphological study of organs and tissues. The animals were dissected immediately upon their death, according to a full pathoanatomical scheme.

Morphometric organs parameters were estimated using Sartorius scales (Germany) with further calculation of organs weights and their standard deviations.

For pathomorphological studies, fresh tissue samples were gelatinated and cryostated to obtain sections up to 5 µm thick. The tissues then were fixed and stained with hematoxylin-eosin. Microscopical investigation was performed with an Opton microscope (Germany).

The experimental data were compared to the reference data and analyzed using statistic software.

### Examination Results

### Local Irritative Effect Estimate

### In-life biomicroscopic study

During the entire observation period the animals preserved normal behavior; hyperemia, oedema, and other changes at sites of injection were not detected.

*Reference intact group.* Skin covers at sites of injection were rose-pink, without oedemas and hyperemia; hair of normal thickness, with healthy gloss without excessive sweat and oil secretion. During observation, defects were not found on epidermis. Reflexes fell within physiological range.

Confocal microscopy: epidermic layer and derma itself had a densely packed structure of terminally differentiated cells. Cells have gap junctions.

*In the therapeutic dose group.* Skin covers at sites of injection were rose-pink, without oedemas and hyperemia; hair of normal thickness, with healthy gloss without excessive sweat and oil secretion. During observation, defects were not found on epidermis. Reflexes fell within physiological range. Confocal microscopy: epidermic layer and derma itself had a densely packed structure of terminally differentiated cells. Cells have gap junctions. No pathological changes were detected.

*In group x10.* No pathological skin changes were detected.

### Clinical and Biochemical studies

Blood analysis: haemoglobin contents, red blood cells, white blood cells, thrombocyte counts in all groups fell within physiological range. Experimental groups data had no significant difference from reference intact groups data and that of the reference substance.

*Biochemical study:* blood glucose, alanine and asparaginic aminotransferase activity in all groups fell within physiological range.

Conclusion. Toxicity study of the original pharmcomposition in subchronic experiment was performed to immature animals.

Intracutaneous introduction both in therapeutic and tenfold dose during two weeks did not result in internal organs changes, nor had a toxic effect on hemic system, nor local irritative effect.

### Example 6. Mutagenicity of the Pharmcomposition

The following set of tests was used to assess mutagenic properties of the claimed protein/polypeptide composition [12]:
- gene mutation account in Ames test of Salmonella / microsome using Salmonella typhimurium TA 97, TA 98 and TA 100 strains as test objects;
- micronuclei account in mice marrow cells.

A sample of protein/polypeptide composition was tested, which was transparent liquid, bottled in a sterile dark glass bottle in the amount of 20 ml, containing the substance in 0.1 mg/ml concentration.

The protein/polypeptide composition mutagenic properties assessment in Ames test.

Mutational test on Salmonella typhimurium is a bacterial test system for account of gene mutation to histidine prototrophy when subjected to chemical compounds and/or their metabolites, inducing base substitution or reading frame shift mutations in this organism genome. Mutagens inducing base pair substitution are agents inducing base pair substitution mutations in DNA molecule. Mutagens inducing genetic code reading frame shift mutations are agents inducing excess or deficiency of single or multiple base pairs in DNA molecule.

This method is used to detect the ability of pharmaceutical substances or their metabolites to induce gene mutations in Salmonella typhimurium indicator strains. The bacteria are treated with the compound being tested with metabolic activation system (CM+) or without metabolic activation (CM-). Upon incubation for a certain period, the number of revertant is counted in different test strains in comparison to the number of spontaneous revertants in negative control options (untreated cultures or solvent treated cultures). If a compound and/or its metabolites being tested are mutagenically active, they will induce back mutations from auxotrophy to histidine prototrophy in histidine-dependent Salmonella typhimurium strains.

For testing, a set of Salmonella typhimurium indicator strains was used, allowing for recording reading frame shift (TA 98 and TA 97) and base substitution (TA 100) mutations.

Strains were taken from Russian National Collection of Industrial Microorganisms of Research Institute for Genetics and Selection of Industrial Microorganisms («Genetika»).

The procedure for handling bacterial cultures, strain genotype check, regulations for their museum storage, necessary experimental equipment, labware, chemical agents, nutritional media and solutions, preparation of rat liver homogenate and activating mixture, and findings statistical analysis methods complied with standards as described in reference literature.

For metabolic activation, the S9 Wistar male rats liver fraction was used; 5 days prior to sacrifice the rats were subjected to microsomal enzymes inducer introduction - sovol (300 mg/kg, single dose, intraperitoneally). In order to monitor the metabolic activation system, ethidium bromide was used (10 µg/dish) on TA 98 strain at CM+.

The protein/polypeptide pharmcomposition in form of sterile solution containing 0.1mg/ml protein was studied: 1.0 and 0.3 ml of original solution per dish and three tenfold dilutions in physiological solution (0.1ml/dish). The substance test doses were 300; 100; 10; 1 and 0.1 µg/dish.

The experiment was supported with positive controls using substances inducing mutations in corresponding tester strains, with and without activation conditions. In cases without activation, sodium azide was used in the amount of 10 µg/dish for TA 100 strain at CM-; 2,7- diamino -4,9- dioxy -5,10- dioxo-4,5,9,10-tetrahydro-4,9- diazopyrene (DIAM) - µg/dish for TA 98 strain at CM-; 9-aminoacridine (9AA) - 50 µg/dish for TA 97 strain at CM-. In order to monitor the metabolic activation system, ethidium bromide was used - µg/dish on TA 98 strain at CM+. As a negative control, physiological solution was used (0.1 ml per dish).

Selective semi-enriched agar (0.7%) in test tubes was melted on boiling-water bath at 100° C and placed to a temperature-controlled water bath at +45 - +46° C.

First, to test tubes with agar 0.1 ml of sample was applied in required dilution concentration, then - 0.1 ml of bacteria suspension and 0.5 ml of microsomal activating mixture (for metabolic activation option). Then the tube content was quickly stirred and plated out to the lower minimum agar layer in Petri dish. Duration of microsomal activating mixture application and semisolid agar plating onto the dish did not exceed 10-15 seconds. The dishes were left at room temperature for 30-40 minutes and after full solidifying were placed to a thermostat at +37°C. The results were recorded after 48 hours of incubation.

Concurrently, the experiment included options without a metabolic activation system (CM-) and with a metabolic activation system (CM+). In the CM- option, direct mutagens effect can be recorded due to the original substance structure activity. Meanwhile, the effect of promutagens, i.e. compounds whose effect is related to formation of mutagenic metabolites, can be taken into account in comparing CM- and CM+ options of the substance study analysis findings. In each reference and experimental option 2 dishes were used. The mutagenic effect was considered significant if the average number of revertant colonies per dish in the experiment option twice and more exceeded that of the reference option. The experiment results were taken into account provided there was a standard response in all options of positive and negative control. The number of revertant colonies in control with solvent in options CM- and CM+ fell within the range of spontaneous level variations for these strains. The strains response to standard mutagens fell within the range of ordinary levels.

The studied protein/polypeptide pharmcomposition in all concentrations tested showed no mutagenic effect on TA 100, TA 98 and TA 97 strains in options with and without metabolic activation system.

CONCLUSION: the protein/polypeptide pharmcomposition does not induce gene mutations in Salmonella typhimurium test strains within the entire range of tested concentrations.

The protein/polypeptide pharmcomposition mutagenic properties assessment by micronucleus method in mammalian cells.

Cytogenetic activity is the ability of a substance to cause structural and numeral chromosomal distortions in somatic and germ cells.

Micronuclei are small DNA-containing formations consisting of acentric chromosome fragments or ana-telophase laggards. At telophase stage, these fragments can be incorporated into daughter cell nuclei or form single or multiple micronuclei in cytoplasm.

The study objective was detection and quantitative estimate of cytogenetic (mutagenic) activity of pharmacologic substances in polychromatophil cells (PCC) of mammal marrow. The method is based on microscopic recording of cells with micronuclei.

The experiments were performed to 2 months old male and female F1 (CBAx C57BI6/J) cross-breed mice weighing 18-20 g. Each group included 6 animals. The animals were kept at 12 hours light pattern with free food and water access. Animal experiments for assessment of mutagenic properties of the protein/polypeptide complex with single and five-time dosing were conducted in compliance with official records. Three sessions of experiments were carried out with corresponding controls: therapeutic dose was tested on male animals with a single introduction; subtoxic dose was tested on male animals with a single introduction; therapeutic dose was tested on male animals with a single introduction; subtoxic dose was tested on male animals and female with five-time introduction.

Taking into account that the possible presumed method for usage of the protein/polypeptide pharmcomposition at a clinic was intravenous or endolumbar introduction, in this study intraperitoneal introduction of the substance to mice was used. The protein/polypeptide complex dosing for assessment of mutagenic activity was calculated based on the maximum human recommended daily therapeutic dose (TD). The protein/polypeptide pharmcomposition was recommended to be introduced in the amount of 2 ml at 0.1 mg/ml concentration. In this case, a human therapeutic dose (TD), including a child's dose, can be 0.05 mg/kg/daily. In order to calculate an experiment dosing for mice, a conversion factor is recommended that takes into account a human or animal body surface area to weight ratio. In our study, it was 8.33. Therefore, the mice TD was approximately 8.33 x human TD (0.42 mg/kg).

As a subtoxic dose, 10mg/kg, the maximum possible in this experiment conditions dosing was used, as the substance was presented in 0.1 mg/ml concentration, and the normally used amount of substance for mice is 0.1 ml per 10 g. Respectively, in conversion to humans, the maximum approved dosing is 1.7mg/kg.

Seven animal groups were formed: four experimental groups and three corresponding reference groups. The substance in the amount of 0.1 mg/kg was introduced five times at 24 hours interval to males and females. All animals were sacrificed 6 hours upon the last introduction of the protein/polypeptide complex.

In two other experimental groups, the protein/polypeptide complex was introduced in single dosing to males in doses of 0.2 mg/kg and 0.5 mg/kg.

Normal saline solvent was used as a negative control. It was introduced intraperitoneally to male and female mice of two reference groups for the same period and in the same amount as to the experimental animals. As a positive control, 20 mg/kg cyclophosphamide was used, diluted ex tempore in normal saline at a single intraperitoneal introduction 24 hours before sacrifice.

Marrow cell preparations for micronuclei count were made by a standard method in compliance with guidelines 
«Оценка мутагенной активности
факторов окружающей среды в клетках разных органов млекопитающих
микроядерным методом»
 ("Environmental Factors Mutagenic Activity Assessment in Various Mammal Organs Cells by the Micronucleus Method"). The animals were sacrificed through cervical dislocation 6 hours after the last introduction. Both thigh bones were detached and muscles were removed from them. In order to wash out the marrow, fetal calf serum was used (produced by NPP PanEko). The marrow from both thigh bones was washed out into a microtube using a syringe. The suspension was centrifugated at 1,000 rpm for 5 min, the supernatant was removed, and the sediment was carefully resuspended. A smear was prepared from a drop of the suspension, which was further air dried out. Then it was fixed with methanol for 3 minutes. Stained according to Romanowsky-Giemsa method. 2,000 polychromatophil cells from each animal were analyzed in encoded preparations. At counting 500 red blood cells, polychromatophilic to normochromatic erythrocytes ratio was defined. Upon analysis completion, the preparations were decoded.

The positive result criterion is the replicable and probably significant increase of micronuclei polychromatophilic erythrocytes number at least in one group compared to the reference group. Achievement of a positive result is indicative of the fact that the substance induces chromosome damage and/or cell mitotic apparatus disturbances in experimental animals.

Micronuclei count statistical analysis was performed by comparing experimental series to reference once by X2 criterion; intergroup comparison of polychromatophilic erythrocytes share was carried out using Mann-Whitney test.

Findings of micronuclei count in mice medulla polychromatophilic erythrocytes: in neither option of the experiment the protein/polypeptide pharmcomposition caused increased frequency of micronucleus cells in mice medulla when comparing experimental and corresponding reference series. Cyclophosphamide (positive control) introduced intraperitoneally to male mice in 20 mg/kg dosage caused increased frequency of micronucleus cells by 19.6 times (62.1 % against 3.16 % in reference, P<0.001). Polychromatophilic erythrocytes share in the total medulla erythrocytes was approximately on the same level in experimental and reference series groups, which is indicative of absence of the pharmcomposition toxic effect on hematogenesis in tested dosing. During application of positive control (cyclophosphamide), polychromatophilic to normochromatic erythrocytes ratio was found to shift to the latter (compared to control P<0.005).

Therefore, the protein/polypeptide pharmcomposition mutagenic activity was not detected in mice medulla polychromatophilic erythrocytes micronuclei induction test at single and five-time dosings introduced intraperitoneally to male and female mice in 0.42 mg/kg dosage, which in conversion is equivalent to a human therapeutic dose. Also, the mutagenic activity was not detected at single introduction of the protein/polypeptide pharmcomposition to males in maximum possible dosing - 10 mg/kg. The protein/polypeptide complex did not show toxic effect by the "polychromatophilic erythrocytes share" criterion.

Conclusion on the protein/polypeptide composition mutagenic properties assessment.

The protein/polypeptide composition does not make a mutagenic effect in Ames test of Salmonella / microsome and in mice medulla polychromatophilic erythrocytes micronuclei induction test in experiments performed in compliance with the Guidelines for Experimental (Non-Clinical) Study of New Pharmaceutical Substances.

### Example 7. Biological Activity Study

Reparative effect. This type of pharmacological effect was established in a standard model with a full thickness skin defect in mice who were subjected to the claimed PPC injections in 0.2 - 1.0 mg/kg dosing. Under the action of the preparation, the healing time reduced from 17 to 10 days (reference healing time). A similar effect is made by Amniocenum, a preparation produced from placenta and introduced in 75 mg/kg dosing. In this respect, a substantial advantage of the claimed preparation is absence of scars in course of healing.

Adaptogenic effect. This type of effect in the process of development of new drugs is becoming one of the most vital areas of research, as it can provide the ability to influence physiological processes at physical, thermal, chemical overloads, especially for avoidance of poisoning, burns, for tolerance enhancement, etc. In course of the claimed preparation testing, it was established that a preliminary treatment of an animal (mouse) for 7 days in a therapeutic dose (0.1 ml/kg) prevents animals from dying of a toxic dose of noxious substances (strychnine), lethal exposure, infecting with blue pus bacillus.

Stress-relieving effect. The antistress effect of the claimed preparation was studied on the basis of multivariable techniques for assessment of condition of different components of emotional stress tolerance in rats (behavioral and visceral components). These studies showed that the claimed preparation can increase emotional stress tolerance, i.e. prevent from higher nervous activity and systemic immunity functional disorders and dysmetabolisms of cardiac function. The most effective dosage was 20 µl per 100 g of a rat weight, which corresponds to 2 ml per 1 injection for a human.

Anticoagulative effect. In course of study of the claimed preparation impact on blood coagulability it was established that in case of introduction of 0.2 mg/kg during one month, the preparation shows ah anticoagulative effect which disappears 30 days after termination of administration. This fact can be of significant practical importance in treatment of conditions with symptomatic hypercoagulability.

### Example 8. Burn-treating and Reparative Effect.

20 F₁ (AMCY x Wistar) rats weighing 250-270 g were anesthetized by intraperitoneal introduction of 60 mg/kg thiopental. In order to make a burn, on a 3x3 cm depilated area in the scapular region a 1cm² four-layered surgical gauze wipe moistened with distilled water was applied, and a 1cm² cautery electrode heated to 200°C was pressed.

The burn degree was regulated by the time of electrode exposure which was 3 to 15 seconds.

The rats were sacrificed on the 3, 7, 14, 23, 30 and 60^{th} day upon making a burn; the burnt tissues were subjected to histological examination. Macroscopic observation showed that immediately after the burn, a sharply marginated dry, dense, off white-grey, with patches of cyanotic color eschar appears on skin. The skin around the thermal influence area becomes oedematous. A pink crown appears around the eschar.

10 rats of the PPC pharmcomposition group received intracutaneous injection in 8 points around the periphery of the burn in the aggregate dose of 0.2 mg/kg, which was 2 ml of the solution. The rest 10 rats formed the reference group receiving intracutaneously 2ml of physiological solution in the same amount of points.

On the third day upon making a burn, a vibrant cell response to the damage appears in the form of lymphomonocytic infiltration around the damaged and unaffected tissues boundary.

On the seventh day, in both groups granulation tissue is found under the eschar, moderately infiltrated with monocytes and lymphocytes, but the experimental group differed from the reference group by the fact that, apart from the ordinary granulation tissue appearing on the damaged area, healing collagen fiber, though thin and delicate, are already aligned with reticular dermis histoarchitecture, and damaged tissues of hypoderm and muscular layer were replaced with loose connective tissue with a slight fibroblast excess. From the boundaries inwards, epidermis was growing under the eschar, with the skin layer structure under the epidermis not different from the reference group. Subepidermic capillaries were enlarged and sanguine in both groups.

By the 14^{th} day, in the pharmcomposition group, under the thin dense eschar and the following narrow area of cell debris, new connective tissue was found, in which thin and weakly fuchsinophil collagen fiber bundles clearly reproduced the reticular dermis collagen fiber bundles structure and architectonics. Under the regenerating derma, there is tissue consisting of a delicate mesh of reticular fibers, between which fat cells are found. This allows to speak of subcutaneous fat structure recovery. Within the reference group, some chaotic nature of collagen fiber bundles arrangement without clear architectonics was observed, under which there was a mesh of reticular fibers with collagen fiber bundles interwoven.

On the 23^{rd} day, in both groups the damaged surface was fully epithelized. Epidermis, slightly thickened on correctly located margins, formed pectiniform outgrowths into subjacent tissues. In the pharmcomposition group, the papillary dermis had closer packed fibers and an increased cellularity compared to the reference group.

On the 30^{th} day of the experiment, further derma collagen fiber maturation was observed in both groups, and in the pharmcomposition, the fibers were slightly smaller with a pronounced structuring. In all animals of the reference group, a significant epidermis thickening was observed.

On the 60^{th} day, the thermal burn area examination showed no visual difference from the subjacent tissues in the pharmcomposition group, there was no qualitative morphological change compared to normal skin tissue. In the reference group, hypertrophic cicatrization was observed with a protuberance over the subjacent skin cover. Morphological examination showed increased content of destructured collagen in all skin layers.

### Example 9. Immunomodulatory activity

Immunomodulatory properties of the PPC produced by the same method as in Examples 1 and 2 were determined by macrophage activation of Wister rats peritoneal exudate in experiments in vitro on enhancement of the enzyme activity in reduction of nitro-blue tetrazolium to diphormazane (NBT-test) and on enhancement of the phagocytic activity to E.Coli.

Into the abdominal cavity of decapitated animals 20 ml of colorless Hanks solution containing 20 U / ml heparin was injected by a syringe with a needle. After a 2-3 minute abdomen massage, the introduced liquid was syringed out of the cavity with the same syringe through an incision, which liquid, after filtration through a nylon filter, was centrifuged at g=150-200 for 15 min. The resulting pellet was suspended in a half volume of a fresh portion of Hanks solution and centrifuged again under the same conditions. Washing of the extrated cells was carried out for 3 - 4 times, after which the cell concentration in the suspension was adjusted to 80 x 10 ⁶ cells / ml. Macrophage content in tissue exudate of all cells was 25-35%. Determination of the enzymatic activity of rat peritoneal exudate macrophages was defined by spectrophotometry using reduction of the nitro blue tetrazolium into diphormazane. The cell sediment of rat peritoneal exudate was diluted with colorless Hanks solution to 80 x 10⁶ cells / ml concentration. To the cell suspension, 50 µl sample with pH=7.4 and the protein-polypeptide complex concentration of 0.1 mg / ml was added. The mixture was incubated on water bath at +37 °C with constant shaking for 30 min, then 50 µl of nitro blue tetrazolium by Reanal (Hungary) solution was added, saturated at 20° C, and incubation was continued for another 30 min. Then 3 ml of acetone was added and centrifuged at 2,000 g for 20 min. The supernatant acetone extract was scanned photometrically at a wavelength of 515 nm. As a control for comparison of enzymatic activity, a sample with a saline-based preservative was used. Phagocytic activity of rat peritoneal exudate cells was determined by their ability to capture the E.Coli. To 50 µl of cell suspension was added 50µl sample with pH=7.4 and the preparation with the total protein concentration of 0.05 mg / ml was added to 50 µl of the cell suspension. The mixture was incubated on water bath at +37 °C with constant shaking for 30 min. Upon incubation, the mixture was suspended in 10 +ml of colorless Hanks solution at +4 °C and centrifuged at 150-200 g for 15 min. The cell sediment of rat peritoneal exudate was diluted with Hanks solution to a concentration of 2.5 x 10 ⁶ cells / ml. The resulting slurry in the amount of 0.2 ml was applied to an 18 x 18 mm piece of glass and incubated at +37 ° C for 30 min in an atmosphere containing 5% carbon dioxide (CO₂) for attaching macrophages to the glass. Upon incubation, for removal of unattached cells from the glass, the latter was rinsed three times in cups containing Hanks medium. To the cells remaining on the glass, 0.2 ml of E.Coli suspension with concentration of 20 x 10⁶ cells / ml was added and incubated under the same conditions for 45min. Subsequently, the glass was rinsed again three times, fixed with methanol and stained according to Romanowsky-Giemsa method. Phagocytosis index (PI) was determined using the following formula: PI (0 + 2,5 n + 6T + 9p + 10P) / number of cells with 0 - the number of cells not phagocytizing E.Coli; n - the number of cells that have phagocytized 1-4 E.Coli cells; T - the number of cells that have phagocytized 5-7 E.Coli cells, P - the number of cells that have phagocytized 8-9 E.Coli cells; R - number of cells that have phagocytized more than 10 E.Coli cells. Macrophages were identified by non-specific esterase staining.

Wister rats peritoneal exudate macrophage activation level in experiments in vitro in the preparation group was significantly exceeding that of the reference group with p <0.05 (12 times) in study of enzyme activity increase in reduction of nitro-blue tetrazolium into diphormazane (NBT-test) and 8 times (p <0.01) in increase of phagocytic activity for E.Coli.

### Example 10. Regenerative Activity and Rejuvenescent Effect

Patient L., 41 y.o. The woman applied in autumn complaining of expressed old wrinkles and appearance of new ones after a vacation spent in the South. Examination revealed crow's feet surface wrinkles in lateral angles of eyes, nasal bridge, forehead, expressiveness of nasal labial fold. The face skin is dry and thinned, its turgor is decreased. Expressed grayness and thinness of hair catch the eye. 5 treatment sessions have been run during three weeks with 3 days intervals, consisting of intracutaneous injections of the subject protein-polypeptide complex according to Example 1 and the pharmcomposition with PPC produced according to Example 3 taken in concentration of 0.1 mg/ml and in the dose of 0.4 mg per session. The preparation was introduced intracutaneously, symmetrically, directly into the wrinkle lines in 10 points on each side, 0.2 ml per point. Injections were made symmetrically chequerwise, at equal intervals from each other. As early as the end of the first week, the following results were noted: the face skin acquired a fresh natural look; its tonicity and elasticity were restored. The external canthus lines had virtually disappeared and on the forehead and nasal bridge have significantly smoothed. By the end of the third week, gray hair amount has decreased. The result over the coming months was stable; the patient was satisfied with the treatment, remarking a significant decrease of gray hair and increase of hair thickness. Therefore, the proposed pharmcomposition allows for improving the efficiency of skin aging treatment due to quick restoration of its fibroarchitecture and systemic immunoregulatory effect on the entire organism.

The attached pictures 1-4 show the complex testing and study results on the invention.

Picture 1 shows the protein-polypeptide complex spectrum pattern in the UV-visible range of the spectrum obtained according to Example 1. According to the picture, the protein-polypeptide complex produced according to the claimed method under Example 1 is characterized by the absorption peak at wavelength of 215 ± 5 nm within UV-visible range.

Picture 2 shows denaturing gel electrophoresis of the Example 1. (2) protein-polypeptide complex in 5% polyacrylamide gel with Coomassie brilliant blue staining compared to the standard set of marker proteins (1) having molecular weights ranging from 10 kDa to 250 kDa.

Picture 3 shows Example 2 protein-polypeptide complex UV-spectrum. According to this picture, in the range of 200 to 500 nm wavelengths, the absorption peak is observed at wavelength of ∼(215±5) nm.

Picture 4 shows denaturing gel electrophoresis of the Example 2. (2) protein-polypeptide complex in 5% polyacrylamide gel with Coomassie brilliant blue staining compared to the standard set of marker proteins (1) having molecular weights ranging from 10 kDa to 250 kDa.

The invention allows for creation of a biologically active substance with enhanced properties and high biological activity having reparative and regenerative tissue-specific and rejuvenating action on skin tissue, and for developing a method for producing the same by extracting biologically active protein-polypeptide complex from nervous and skin tissues ungulate farm embryos which is solved by a selected set of procedures and modes. Particularly important was to determine the optimal gestation timing of fetal skin and nervous tissues, as well as a set of reagents, their dosage, extraction and buffering regimes, to maintain effective concentration ratios of proteins, polypeptides, and nucleotides fixed evolutionarily and defining the biological activity of the complex, providing reparative and regenerative tissue-specific and rejuvenating effect.

### Industrial Applicability

The invention is intended for use in chemical and pharmaceutical industries, medicine and cosmetics, is related to biologically active substance - protein/polypeptide complex derived from embryonic neural and skin tissues of hoofed farm animals, having antihypoxic, rejuvenating effect, stimulating physiological and reparative regeneration of skin tissue, for use in production of medicines and cosmetics for skin diseases, aesthetic medicine, and cosmetology.

Sources of information taken into account during preparation of the application:
1. RF Patent No.2355405, IPC A61 K39/17, A61K35/50
2. Application of France No. 2413912
3. RF Patent No.2132688 IPC A61 K35/48
4. Patent No. RU 2289417 C1
5. RF Patent No.2189257, I PC A 61 L 27/00, A 01 N 1/00)
6. RF Patent No.2142784 IPC A61 K7/48, A61 K35/78)
7. RF Patent No.2144815, C1(51) A61K7/00, A61 K7/48, A61P17/16
8. US Patent No.9703169
9. Application of France No. 2530466, IPC A 61 K 7/48, 27.01.2084
10. RF Patent No.2229886 C1
11. International Application WO 2009/088314 A1
12. 
Руководство по экспериментальному (доклиническому)
изучению новых фармакологических веществ. Под общей
редакцией чл.-кор. РАМН, проф. Р.У. Хабриева.- 2 изд., перераб. и
доп. -М, ОАО Издательство «Медицина»
 - 2005., c.131-170. (Guidelines for Experimental (Non-Clinical) Study of New Pharmaceutical Substances. Under the general editorship of Associate Member of RAMS professor R.U.Khabriyev. - 2nd edition, revised and enlarged - M., OAO Izdatelstvo Meditsyna - 2005, pages 131-170)
13. 
Методические указания по оценке мутагенных свойств
фармакологических веществ // Руководство по экспериментальному
(доклиническому) изучению новых фармакологических веществ. Под
общей редакцией чл.-кор. РАМН, проф. Р.У. Хабриева.- 2 изд.,
перераб. и доп. -М, ОАО Издательство «Медицина»
 - 2005., c.100-122. (Instructional Guidelines for Pharmaceutical Substances Mutagenic Properties Assessment // Guidelines for Experimental (Non-Clinical) Study of New Pharmaceutical Substances. Under the general editorship of Associate Member of RAMS professor R.U.Khabriyev. - 2nd edition, revised and enlarged - M., OAO Izdatelstvo Meditsyna - 2005, pages 100-122)
14. 
Методические указания по оценке канцерогенности свойств
фармакологических средств и вспомогательных веществ в
краткосрочных тестах.// Руководство по экспериментальному
(доклиническому) изучению новых фармакологических веществ. Под
общей редакцией чл.-кор. РАМН, проф. Р.У. Хабриева.- 2 изд.,
перераб. и доп. -М, ОАО Издательство «Медицина»
 - 2005., c.131-170. (Instructional Guidelines for Pharmaceutical Substances and Excipients Carcinogenic Potency in Short-Term Tests.// Guidelines for Experimental (Non-Clinical) Study of New Pharmaceutical Substances. Under the general editorship of Associate Member of RAMS professor R.U.Khabriyev. - 2nd edition, revised and enlarged - M., OAO Izdatelstvo Meditsyna - 2005, pages 131-170)
15. 
Программа по уходу и содержанию лабораторных животных
НПП «Питомник лабораторных животных» ФИБХ РАН от ноября
 2005 r. (A Program for Care and Management of Laboratory Animals of the Nursery for laboratory animals of the Institute of Bioorganic Chemistry of the Russian Academy of Sciences, dated November, 2005)

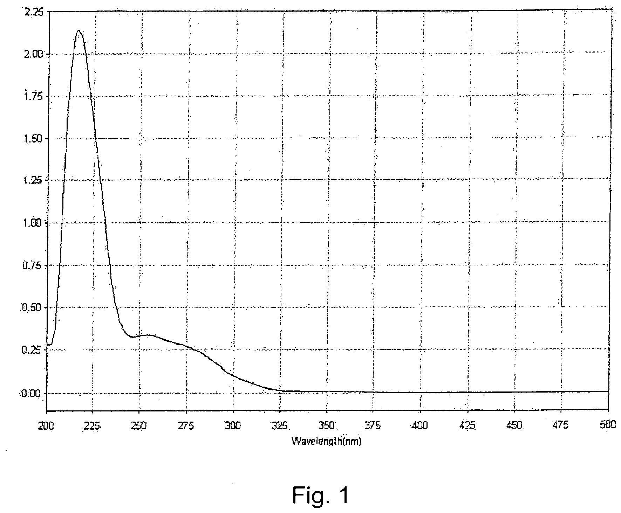

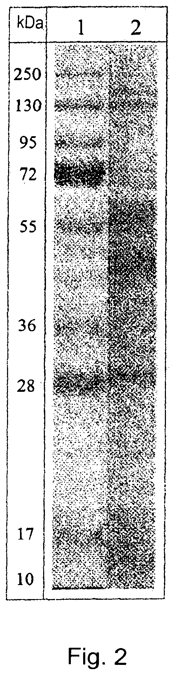

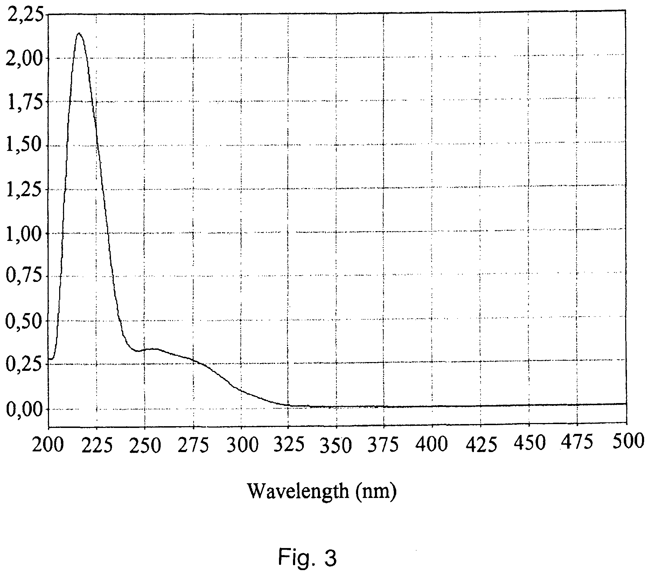

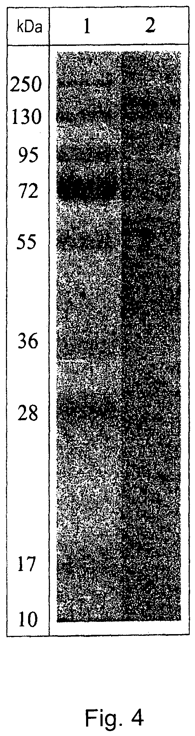

## Claims

1. A method for producing the protein/polypeptide complex, in which nerve and skin tissues of hoofed farm embryos of gestation stage from the middle of the first third to the middle of the last third of pregnancy are stepwise:
- homogenized in a buffer at the buffer: tissue ratio of at least 1:0.5, with a simultaneous extraction in presence of nonionic detergents at minimum pH 5.2 and maximum pH 8.5, with further centrifugation at g ranging from 10 000 - 28 000 during 90 - 30 min;
- the resulting supernatant is filtered;
- the filtrate undergoes anion-exchange chromatography on a column with an anion-exchange carrier and separating proteins and polypeptides associated with the carrier, using for a mobile phase a buffer solution initially having ionic strength (I) not exceeding 0.1 mmol/L increasing it smoothly or stepwise with 0.025 mmol/L increment and starting to collect target fractions with eluent ionic strength ranging from 0.125 to 0.15 mmol/L at pH from 5.2 to 8;
- the resulting fractions are associated and undergo sterilization filtration.

2. A protein/polypeptide complex (PPC) produced using the method according to Claim 1 produced from extracted homogenate of nerve and skin tissues of hoofed farm embryos of gestation stage from the middle of the first third to the middle of the last third of pregnancy, including negatively charged faintly acid, neutral proteins and polypeptides, with molecular weights ranging from 5 to 200 kDa, therewith at least 70% of the total protein amount having molecular weights ranging from 20 to 160 kDa, **characterized by** solution ultraviolet spectrum absorption peak at wavelength 215±5 nm, a peak recorded at wavelengths from 200 - 500 nm in the UV-visible range of the spectrum and the presence of specific bands at denaturing gel electrophoresis ("SDS-Page") in 5% polyacrylamide gel compared to the standard set of marker proteins having molecular weights ranging from 10 kDa to 250 kDa and an absorption peak at wavelength of 215±5 nm at ultraviolet spectrum recorded at wavelengths ranging from 200 to 500 nm.

3. A PPC-based pharmaceutical composition, wherein the composition is prepared as a solution containing, as an active ingredient, the biologically active protein/polypeptide complex according to Claim 2 in concentration of 0.05 - 2.0 mg/ml, obtainable by the method according to Claim 1, and a pharmaceutically acceptable diluent.

4. A pharmaceutical composition according to Claim 3, **characterized in that** it contains a pharmacopoeial buffer as a pharmaceutically acceptable diluent and excipients including high-molecular compounds, stabilizers, preservatives and solubilizers.

5. A pharmaceutical composition according to Claim 3, **characterized in that** it is formulated for parenteral - intradermal or subcutaneous, intranasal or contact administration to mucous membranes and skin.

## Patentansprüche

1. Verfahren zur Herstellung des Protein/Polypeptid-Komplexes, in welchem Nerven- und Hautgewebe von Huftierzucht-Embryos des Trächtigkeitsstadiums von der Mitte des ersten Drittels bis zur Mitte des letzten Drittels der Schwangerschaft schrittweise
- in einem Puffer bei dem Verhältnis Puffer: Gewebe von mindestens 1:0,5 homogenisiert werden, mit einer gleichzeitigen Extraktion in Gegenwart nichtionischer Detergenzien bei einem minimalem pH 5,2 und maximalem pH 8,5, weiterhin mit Zentrifugation bei g im Bereich von 10 000 - 28 000 während 90-30 Minuten;
- der resultierende Überstand wird filtriert;
- das Filtrat wird einer Anionenaustauschchromatographie auf einer Säule mit Anionenaustauschträger ausgesetzt, und Auftrennen von Proteinen und Polypeptiden, welche mit dem Träger verbunden sind, unter Verwendung einer Pufferlösung als eine mobile Phase, die zu Beginn eine Ionenstärke (I) von nicht höher als 0,1 mmol/L aufweist, Steigern dieser gleichmäßig oder schrittweise mit einem Inkrement von 0,025 mmol/L, und Beginnen, Zielfraktionen mit Eluenten mit einer Ionenstärke von 0,125 bis 0,15 mmol/L bei einem pH von 5,2 bis 8 zu sammeln;
- die resultierenden Fraktionen werden vereint und einer Sterilfiltration ausgesetzt.

2. Protein/Polypeptid-Komplex (PPC), hergestellt unter Verwendung des Verfahrens nach Anspruch 1, hergestellt aus extrahiertem Homogenisat von Nerven- und Hautgewebe von Huftierzucht-Embryos des Trächtigkeitsstadiums von der Mitte des ersten Drittels bis zur Mitte des letzten Drittels der Schwangerschaft, beinhaltend negativ geladene, leicht saure, neutrale Proteine und Polypeptide, mit Molgewichten im Bereich von 5 bis 200 kDa, damit mindestens 70% der gesamten Proteinmenge mit Molgewicht im Bereich von 20 - 160 kDa, charakterisiert durch Lösung ultraviolettes Spektrum Absorptionsmaximum bei Wellenlänge 215±5 nm, einem Maximum aufgenommen bei Wellenlängen von 200 - 500 nm in dem UV-sichtbaren Bereich des Spektrums und der Anwesenheit bestimmter Banden in denaturierender Gelelektrophorese ("SDS-Page") in 5% Polyacrylamidgel, verglichen mit dem Standardsatz von Referenzproteinen mit Molgewichten im Bereich von 10 kDa bis 250 kDa, und ein Absorptionsmaximum bei Wellenlänge 215±5 nm im ultravioletten Spektrum, aufgenommen bei Wellenlängen in dem Bereich von 200 bis 500 nm.

3. Pharmazeutische Zusammensetzung, auf PPC basierend, wobei die Zusammensetzung als eine Lösung hergestellt wird, enthaltend als einen aktiven Inhaltsstoff in einer Konzentration von 0,05 - 2,0 mg/mL den biologisch aktiven Protein/Polypeptid-Komplex nach Anspruch 2, erhältlich durch das Verfahren nach Anspruch 1, und ein pharmazeutisch akzeptables Verdünnungsmittel.

4. Pharmazeutische Zusammensetzung nach Anspruch 3, **dadurch gekennzeichnet, dass** sie einen Puffer aus einem Arzneibuch als pharmazeutisch akzeptables Verdünnungsmittel und Hilfsstoffe, inklusive hochmolekularer Verbindungen, Stabilisatoren, Konservierungsmittel und Lösungsvermittler, enthält.

5. Pharmazeutische Zusammensetzung nach Anspruch 3, **dadurch gekennzeichnet, dass** sie für parenteral formuliert ist - intradermal oder subkutan, intranasal oder Kontaktverabreichung auf Schleimhäute und Haut.

## Revendications

1. Procédé de production du complexe protéine/ polypeptide, dans lequel les tissus nerveux et de peau d'embryons de ferme d'ongulés de stade de gestation du milieu du premier tiers au milieu du dernier tiers de grossesse sont par étape :
- homogénéisés dans un tampon au rapport tampon:tissu d'au moins 1:0,5, avec une extraction simultanée en présence de détergents non ioniques au minimum à pH 5,2 et au maximum à pH 8,5, avec en outre une centrifugation à g se situant dans la plage allant de 10 000 à 28000 durant 90 à 30 minutes ;
- le surnageant obtenu est filtré ;
- le filtrat subit une chromatographie d'échange d'anions sur une colonne avec un support d'échange d'anions et la séparation des protéines et des polypeptides associés avec le support, en utilisant pour phase mobile une solution de tampon ayant initialement une force ionique (I) ne dépassant pas 0,1 mmol/l en l'augmentant doucement ou par étape avec un incrément de 0,025 mmol/l et en commençant à collecter les fractions cibles avec une force ionique d'éluant se situant dans la plage allant de 0,125 à 0,15 mmol/l à un pH de 5,2 à 8 ;
- les fractions obtenues sont associées et subissent une filtration de stérilisation.

2. Complexe protéine/polypeptide (PPC) produit en utilisant le procédé selon la revendication 1 d'un homogénat extrait de tissus nerveux et de peau d'embryons de ferme d'ongulés de stade de gestation du milieu de premier tiers au milieu de dernier tiers de la grossesse, comprenant un acide légèrement chargé négativement, des protéines neutres et des polypeptides, avec des poids moléculaires se situant dans la plage allant de 5 à 200 kDa, avec au moins 70 % de la quantité de protéines totale ayant des poids moléculaires se situant dans la plage allant de 20 à 160 kDa, **caractérisés par** un pic d'absorption de spectre ultraviolet en solution à une longueur d'onde de 215 ± 5 nm, un pic enregistré à des longueurs d'onde de 200 à 500 nm dans la plage UV-visible du spectre et la présence de bandes spécifiques en électrophorèse sur gel dénaturant (« SDS-Page ») dans un gel à 5 % de polyacrylamide par rapport au jeu standard de protéines marqueurs ayant des poids moléculaires se situant dans la plage allant de 10 à 250 kDa et un pic d'absorption à la longueur d'onde de 215 ± 5 nm à un spectre ultraviolet enregistré à des longueurs d'onde se situant dans la plage allant de 200 à 500 nm.

3. Composition pharmaceutique à base de PPC, la composition étant préparée comme une solution contenant, comme ingrédient actif, le complexe protéine/polypeptide biologiquement actif selon la revendication 2 à une concentration de 0,05 à 2,0 mg/ml, que l'on peut obtenir par le procédé selon la revendication 1, et un diluant pharmaceutiquement acceptable.

4. Composition pharmaceutique selon la revendication 3, **caractérisée en ce qu'**elle contient un tampon de pharmacopée comme diluant pharmaceutiquement acceptable et des excipients comprenant des composés de haut poids moléculaire, des stabilisants, des conservateurs et des solubilisants.

5. Composition pharmaceutique selon la revendication 3, **caractérisée en ce qu'**elle est formulée pour l'administration parentérale - intradermique ou sous-cutanée, intranasale ou de contact à des membranes muqueuses et à la peau.
